(19) Europäisches Patentamt European Patent Office Office européen des brevets

(11) **EP 3 988 633 B1**

(12) **EUROPÄISCHE PATENTSCHRIFT**

(45) Veröffentlichungstag und Bekanntmachung des Hinweises auf die Patenterteilung:
**27.09.2023   Patentblatt 2023/39**

(21) Anmeldenummer: **21157181.5**

(22) Anmeldetag: **15.02.2021**

(51) Internationale Patentklassifikation (IPC):
***C10L 3/10*** *(2006.01)*

(52) Gemeinsame Patentklassifikation (CPC):
**C10L 3/101; C10L 3/104;** C10L 2290/548;
Y02C 20/40; Y02P 20/133

(54) **POLYMER-TRENNMEMBRANEN ZUR REINIGUNG VON METHAN**

POLYMER SEPARATION MEMBRANES FOR METHANE PURIFICATION

MEMBRANES DE SÉPARATION EN POLYMÈRE DESTINÉES À LA PURIFICATION DU MÉTHANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.10.2020   AT 603152020**

(43) Veröffentlichungstag der Anmeldung:
**27.04.2022   Patentblatt 2022/17**

(73) Patentinhaber: **Axiom Angewandte Prozeßtechnik Ges. m.b.H.**
**2483 Ebreichsdorf (AT)**

(72) Erfinder: **Makaruk, Aleksander**
**1050 Wien (AT)**

(74) Vertreter: **Ellmeyer, Wolfgang**
**Patentanwalt**
**Mariahilferstrasse 50**
**1070 Wien (AT)**

(56) Entgegenhaltungen:
**WO-A1-2015/017875     US-A1- 2012 323 059**

**Beschreibung**

Die vorliegende Erfindung betrifft die Verwendung von Polymer-Trennmembranen zur Reinigung von durch Methanisierung erhaltenem Methan.

STAND DER TECHNIK

Methanisierung, also die Produktion von - auch als "synthetic natural gas" (kurz SNG) bezeichnetem - Methan durch Hydrierung von Kohlenstoffmonoxid und -dioxid, gewinnt in den letzten Jahren stetig an Bedeutung, da sich aufgrund des Klimawandels und der schwindenden Vorräte an fossilen Ressourcen die Energieversorgung zunehmend in Richtung erneuerbarer Energiequellen verlagert. Speziell die Methanisierung unter Verwendung von $CO_2$ aus der Atmosphäre, die aufgrund des geringen $CO_2$-Gehalts der Luft (rund 400 ppm) und des hohen Energieaufwands chemischer Trennverfahren vor Kurzem noch als ineffizient und im industriellen Maßstab kaum umsetzbar galt, gerät immer stärker in den Fokus von Verfahrenstechnikern. Da auch der als Reaktionspartner benötigte Wasserstoff inzwischen in zunehmendem Maße auf nachhaltige Weise durch Wasserelektrolyse mit aus Wind- und Solarkraft gewonnenem Strom erzeugt wird (wobei das durch die Methanisierung gewonnen $CH_4$ auch als "Windgas" bzw. "Solargas" bezeichnet wird), werden auf diesem Gebiet laufend verbesserte Verfahren entwickelt.

Ein Hauptaugenmerk liegt dabei auch auf der Verwendung von durch Methanisierung gewonnenem Methan als synthetischer Treibstoff für so genannte "Erdgasfahrzeuge". Zwar sind Elektro- und Hybridfahrzeuge derzeit noch stark im Aufschwung begriffen, allerdings gilt der Elektroantrieb in Fachkreisen nicht unbedingt als die Technologie der Zukunft. Im Falle einer Produktion solcher Fahrzeuge in deutlich größerem Maßstab droht nämlich ebenfalls eine Rohstoffverkknappung, z.B. von Lithium und Cobalt sowie bestimmter Seltener Erden, und zudem sind die Speicherkapazität, Lebensdauer und Zyklenfestigkeit der Antriebsbatterien weiterhin relativ begrenzt.

Bei Methanisierungsverfahren unter Nutzung von Kohlenstoffdioxid aus der Atmosphäre zur Produktion von Methan zur Verwendung als synthetischer Treibstoff sind jedoch hohe Anforderungen an die Reinheit sowohl des aus der Luft abgeschiedenen $CO_2$ als auch des dabei erhaltenen Methans zu stellen. Beispielsweise liegen bei Einspeisung des Methans in das Erdgasnetz die Grenzwerte für die Konzentrationen von $H_2$ und $CO_2$ darin im einstelligen Prozentbereich, z.B. gemäß ÖVGW-Richtlinie G31 bei maximal 4 Vol.-% $H_2$ und 2 Vol.-% $CO_2$. In letzter Zeit gibt es jedoch Bestrebungen, diese Grenzwerte insofern anzupassen, als die Grenze des Korrosionseffekte bewirkenden $CO_2$ noch weiter gesenkt, z.B. unter 1 Vol.-% oder sogar unter 0,5 Vol.-%, jene des den Brennwert des Erdgases steigernden $H_2$ hingegen erhöht werden soll, z.B. auf 10 Vol.-%.

Allerdings läuft die Methanisierung von Kohlenstoffdioxid gemäß der allgemeinen Reaktionsgleichung

$$4 H_2 + CO_2 \rightarrow CH_4 + 2 H_2O$$

unter technischen Bedingungen niemals vollständig ab, so dass das Produktgasgemisch neben $CH_4$ und $H_2O$ mitunter erhebliche Mengen der nichtumgesetzten Ausgangsprodukte $H_2$ und $CO_2$ umfasst. Diese sind nicht nur bei den meisten Anwendungen des gewonnenen Methans unerwünscht, sondern können zudem zur Methanisierung rezykliert werden. Aus letzterem Grund wird speziell bei der katalytischen Methanisierung von (in der Umgebungsluft enthaltenem) $CO_2$ in der Regel ein Überschuss an $H_2$ eingesetzt, um den Umsatz des $CO_2$ zu $CH_4$ zu erhöhen, wonach der überschüssige Wasserstoff rezykliert wird.

Weiters entstehen während der Methanisierungsreaktion als Nebenprodukte üblicherweise auch höhere Kohlenwasserstoffe, vor allem solche mit zwei bis vier Kohlenstoffatomen, die jedoch keine unerwünschten Komponenten darstellen, da sie den spezifischen Brennwert des Gasgemischs erhöhen und sich bei der Membrantrennung ähnlich wie Methan verhalten und gemeinsam damit gereinigt werden können.

Zur Trennung des Methans von anderen Neben- und nichtumgesetzten Ausgangsprodukten werden neben Kryo-, Adsorptions- (z.B. Druckwechseladsorption) und Absorptionsverfahren häufig auch Membrantrennverfahren eingesetzt. Beispielsweise offenbart die Technische Universität Wien in WO 2015/017875 A1 ein Verfahren zum Speichern von Energie, in dem durch Wasserelektrolyse $H_2$ und davon getrennt $CO_2$ erzeugt werden, aus denen in weiterer Folge durch Methanisierung $CH_4$ gebildet wird, das in der Folge in ein Erdgasnetz eingespeist werden kann. Zur Reinigung des Produktgases der Methanisierung wird ein Membrantrennsystem unter Verwendung von Gastrennmembranen offenbart, die zu einer selektiven Abtrennung von $CO_2$ und $H_2$ und gegebenenfalls auch von $H_2O$ vom erzeugten $CH_4$ in der Lage sind, wofür Polymerfilm-, Metall- und Keramikmembranen als geeignet offenbart werden. Vorzugsweise erfolgt die Gastrennung jedoch in einem einzigen Membrantrennschritt, d.h. mittels Membranen, die für alle drei zu entfernenden Gase eine höhere Selektivität aufweisen als für $CH_4$. Zu diesem Zweck werden Membranen aus Kunststoff, insbesondere Polyimid-Membranen offenbart, für die eine Selektivität bei der Trennung $H_2/CH_4$ von 60 und für $CO_2/CH_4$ von 20 offenbart wird. Für Polyimid-Membranen wird zudem eine Selektivität für die Trennung $H_2O/CH_4$ von über 100 bis hin zu 1000 offenbart.

Die Selektivität wird dabei als Parameter α angegeben, wobei es sich um die so genannte ideale Selektivität für ein Gaspaar handelt, d.h. um das Verhältnis der Permeabilitäten P der beiden Gaskomponenten für einen bestimmten Membrantyp. Für die Zwecke der vorliegenden Erfindung werden diese hierin in der Folge als α1, α2 bzw. α3 gemäß den nachstehenden Formeln bezeichnet:

$$\alpha 1 = \frac{P_{\text{H2}}}{P_{\text{CH4}}} \qquad \alpha 2 = \frac{P_{\text{CO2}}}{P_{\text{CH4}}} \qquad \alpha 3 = \frac{P_{\text{H2O}}}{P_{\text{CH4}}}$$

Dazu offenbaren Tanihara et al., J. Membr. Sci, 160, 179-186 (1999), für aus Biphenyltetracarbonsäuredianhydrid (BPDA) und verschiedenen aromatischen Diaminen hergestellte Polyimide für die Trennungen $H_2/CH_4$ und $CO_2/CH_4$ bei 50 °C die Selektivitäten α1 = 130 und α2 = 40, während Yang et al., Polymer 42, 2021-2029 (2001), für ein aus 4,4'-(Hexafluorisopropyliden)diphthalsäureanhydrid (6FDA) und 2,6-Dimethyl-3,7-diaminodibenzothiophen-5,5-dioxid (DBBT) hergestelltes Polyimid die Werte α1 = 80 und α2 = 44 (bei 35 °C) offenbaren.

Als weitere Kunststoffmembranen neben Polyimid-Membranen sind zur Reinigung von Methanisierungs-Produktgas beispielsweise auch Polysulfon- und Celluloseacetat-Membranen bekannt, da sie ähnlich hohe Selektivitäten für die Trennungen $H_2/CH_4$ und $CO_2/CH_4$ aufweisen, mit denen die Gasmembrantrennung sehr effizient betrieben werden kann, da sie hohe Gasausbeuten und -reinheiten sowie eine relativ geringe Rekompressionsleistung gewährleisten.

Unter Rekompressionsleistung ist hierin der Energieaufwand zu verstehen, der erforderlich ist, um das zum Reaktor rezyklierte, an $CH_4$ abgereicherte Permeat der Gasmembrantrennung mit dem bei der Methanisierung herrschenden Druck zu beaufschlagen. Dieser beträgt üblicherweise einige bis hin zu mehreren Dutzend Bar, mitunter sogar 100 bar oder mehr, um das Gleichgewicht der Methanisierungsreaktion gemäß dem Prinzip von Le Chatelier und Braun zur Produktseite hin zu verschieben, da sich bei der Reaktion das Gasvolumen verringert (aus fünf Molekülen Edukte werden drei Moleküle Produkte).

Die Selektivität der Trennung $H_2/CH_4$ unter Verwendung von Membranen nach dem Stand der Technik wie etwa Polyimid-, Polysulfon- und Celluloseacetat-Membranen ist dabei durchwegs höher als bei $CO_2/CH_4$, d.h. α1 > α2 bzw. α1/α2 > 1, was auch den Verbrauch von im Überschuss eingesetztem und anschließend rezykliertem $H_2$ verringert. Wie freilich bereits zuvor aus WO 2015/ 017875 A1 zitiert wurde, ist die Selektivität der Trennung $H_2O/CH_4$, d.h. der Wert für α3, im Allgemeinen am höchsten.

All den genannten Kunststoffmembranmaterialien ist gemein, dass sie bei der jeweiligen Betriebstemperatur der Membrantrennung im glasartigen ("glassy") oder hartelastischen, spröden Zustand vorliegen müssen, um die hohen Selektivitäten bei der Gastrennung erreichen zu können. Aufgrund dessen werden bevorzugt Membranmaterialien eingesetzt, die relativ hohe Anteile an aromatischen Ringen, vor allem an voluminösen Aromaten, in den Polymerketten aufweisen, um für hohe Glasübergangstemperaturen $T_g$ zu sorgen. Auf diese Weise wird vermieden, die Membranen im Trennbetrieb kühlen zu müssen, um die Polymere im glasartigen Zustand unterhalb der Glasübergangstemperatur zu halten. Allerdings sind solche Kunststoffe, darunter vor allem Polyimide mit relativ starren Polymerketten, kaum noch oder gar nicht schmelzbar und in den meisten organischen Lösungsmitteln unlöslich, was ihre Verarbeitung erschwert und verteuert.

Darüber hinaus ist es angesichts der zuvor zitierten Grenzwerte für die Konzentrationen von $H_2$ und $CO_2$ in einem in das Erdgasnetz einzuspeisenden Methanstrom, die für $H_2$ deutlich höher liegen als für $CO_2$, von Nachteil, dass die obigen, zur Membrantrennung eingesetzten Kunststoffmembranen durchwegs eine höhere Selektivität für die Abtrennung von Wasserstoff als für jene von Kohlenstoffdioxid von Methan aufweisen. Aus diesem Grund müssen die Produktgasströme von Methanisierungsverfahren einer größeren Anzahl von Membrantrennschritten bzw. -zyklen unterzogen werden, um den $CO_2$-Gehalt des Methans auf einen zulässigen Wert abzusenken. Das heißt, im kontinuierlichen Betrieb unter Rezyklierung des an $CO_2$ und $H_2$ angereicherten Permeats der Membrantrennung müssen größere Mengen an Permeat rezirkuliert und rekomprimiert werden, was den Energieverbrauch deutlich erhöht, und die Wirtschaftlichkeit des Systems senkt.

Eine weitere Gruppe von Kunststoffmembranen, die häufig zur Gastrennung eingesetzt werden, sind sogenannte Elastomer-Membranen, die im Gegensatz zur den oben beschriebenen Polyimid-Membranen oberhalb ihrer Glasübergangstemperaturen $T_g$, also im gummiartigen ("rubbery") Zustand eingesetzt werden. Diese bestehen zumeist aus Polyethern, wie z.B. Polytetramethylenglykol bzw. Polytetrahydrofuran (PolyTHF), Polyethylenglykol (PEG) und Polypropylenglykol (PPG), oder auch Polyether-Block-Polyamid- (PEBA-) Copolymeren. Diese weisen in der Regel eine - mitunter sogar deutlich - höhere Selektivität für $CO_2$ als für $H_2$ auf, weswegen sich ihr Einsatz zur Abtrennung von $CO_2$ aus Abgasen anbietet.

Beispielsweise offenbaren Li et al., J. Membr. Sci. 369, 49-58 (2011), Permeabilitätsversuche mit Membranen aus PEG, PPG, im Handel unter dem Markennamen Terathane® erhältlichen PolyTHF-Membranen sowie mit Verbundmembranen aus Kombinationen dieser Kunststoffe. Dabei wurden zunächst die Durchlässigkeiten der Membranen für sechs verschiedene Gase, nämlich $O_2$, $N_2$, $H_2$, He, $CH_4$ und $CO_2$, bei unterschiedlichen Drücken untersucht und daraus ihre Selektivitäten für die Abtrennung von $CO_2$ aus binären Gasgemischen berechnet. Für die Trennung $CO_2/CH_4$ ergaben sich dabei Werte (d.h. $\alpha 1$-Werte) von rund 7 und für die Trennung $CO_2/H_2$ von unter 5. Als Anwendungsgebiete solcher Membranen werden die Trennungen $CO_2/H_2$ aus Synthesegas, $CO_2/N_2$ aus der Luft, $CO_2/CH_4$ für die Erdgasreinigung sowie $CO_2/O_2$ bei der Lebensmittelverpackung erwähnt.

Eine andere Arbeitsgruppe offenbart in mehreren Publikationen Untersuchungen zur Permeabilität und Selektivität von PEBA- (unter dem Handelsnamen Pebax® erhältlich) sowie PEBA/PEG-Verbundmembranen, wobei zunächst nur reine Gase (Car et al., J. Membr. Sci. 307, 88-95 (2008)), später aber auch Gasgemische getestet wurden (Car et al., Sep. Purif. Technol. 62, 110-117 (2008)). Dabei wurden im Mittel $\alpha 1$-Werte für die Trennung $CO_2/CH_4$ von etwa 15 und für $CO_2/H_2$ von etwa 10 festgestellt. In letzterem Artikel wird ein möglicher Einsatz dieser Membranen für die Abtrennung von $CO_2$ aus Abgasen, wie z.B. aus Durchlauferhitzern, Kohleverbrennungsanlagen und Ölraffinerien, offenbart.

Und schließlich offenbaren Ahmadpour et al., J. Nat. Gas Sci. Eng. 21, 518-523 (2014), die Verwendung einer PEBA-Membran sowie einer PEBA/PVC-Verbundmembran zur Erdgasreinigung und die Messung der Permeabilitäten dieser Membranen für reines $CO_2$ und $CH_4$ unter Variation des Drucks und der Temperatur, woraus in der Folge wiederum die Selektivitäten $\alpha 1$ berechnet wurden. Dabei wurden Werte im Bereich zwischen 22 und 35 erhalten, wobei jene der Verbundmembran mit PVC kaum besser waren als die Werte für PEBA alleine. Die Durchlässigkeit der Verbundmembran für Wasserstoff wurde nicht bestimmt, wird sich aber anzunehmenderweise ebenfalls nicht allzu stark von jener der PEBA-Membran unterscheiden.

Vor diesem Hintergrund war ein Ziel der Erfindung die Entwicklung eines neuen Verfahrens zur Produktion von Methan durch Reduktion von $CO_2$ mit $H_2$ mit anschließender Membrantrennung des Produktgases, bei dem die obigen Nachteile zumindest teilweise behoben werden können.

OFFENBARUNG DER ERFINDUNG

Dieses Ziel erreicht die vorliegende Erfindung durch Bereitstellung der Verwendung von Polymer-Trennmembranen, die in der Lage sind, $CO_2$ und $H_2$ selektiv von $CH_4$ abzutrennen, in einem Membrantrennschritt zur Reinigung von in einem $CH_4$, $H_2$ und $CO_2$ umfassenden, gegebenenfalls vorgetrockneten, Produktgasgemisch eines Methanisierungsverfahrens enthaltenem Methan, wobei die Verwendung gemäß vorliegender Erfindung dadurch gekennzeichnet ist, dass

a) die Trennung bei einer Betriebstemperatur $T_B$ zwischen -20 °C und 100 °C durchgeführt wird; und
b) die Polymermembranen

b1) in der Lage sind, $CO_2$ und $H_2$ gleichzeitig von $CH_4$ abzutrennen,
b2) eine höhere Selektivität für die Abtrennung von $CO_2$ als für jene von $H_2$ von $CH_4$, d.h. ein Verhältnis $\alpha 1/\alpha 2$ < 1, aufweisen und
b3) eine Glasübergangstemperatur $T_g$ aufweisen, die unter der Betriebstemperatur $T_B$ liegt.

Anders ausgedrückt wird hierin ein Verfahren zur Produktion von Methan bereitgestellt, das die folgenden Schritte umfasst:

einen Methanisierungsschritt, in dem durch Reduktion von $CO_2$ mit $H_2$ ein neben $CH_4$ auch $H_2O$, $H_2$ und $CO_2$ umfassendes Produktgas gebildet wird;
gegebenenfalls einen Trockungsschritt, in dem $H_2O$ aus dem Produktgas entfernt wird; und
einen Membrantrennschritt zur Reinigung des Methans, in dem das durch die Trocknung erhaltene, $CH_4$, $H_2$ und $CO_2$ umfassende Gasgemisch einer Trennung unter Verwendung von Trennmembranen unterzogen wird, die in der Lage sind, $CO_2$ und $H_2$ selektiv von $CH_4$ abzutrennen;

und das dadurch gekennzeichnet ist, dass

a) die Trennung im Membrantrennschritt bei einer Betriebstemperatur $T_B$ zwischen -20 °C und 100 °C durchgeführt wird; und
b) Polymermembranen eingesetzt werden, die

b1) in der Lage sind, $CO_2$ und $H_2$ gleichzeitig von $CH_4$ abzutrennen,
b2) eine höhere Selektivität für die Abtrennung von $CO_2$ als für jene von $H_2$ von $CH_4$, d.h. ein Verhältnis $\alpha 1/\alpha 2$ < 1, aufweisen und

b3) eine Glasübergangstemperatur $T_g$ aufweisen, die unter der Betriebstemperatur $T_B$ liegt.

Durch den Einsatz von Polymermembranen zur Reinigung eines Methanisierungs-Produktgases, die im diametralem Gegensatz zum Stand der Technik $CO_2$ mit höherer Selektivität von $CH_4$ abzutrennen in der Lage sind als $H_2$, d.h. bei denen $\alpha 2 > \alpha 1$ bzw. $\alpha 1/\alpha 2 < 1$ ist, und die oberhalb ihrer Glasübergangstemperaturen, also in ihrem gummiartigen Zustand eingesetzt werden, ist es auf einfachere und kostengünstigere Weise möglich, durch Methanisierung von $CO_2$ und anschließende Membranreinigung Methan bereitzustellen, das für die Einspeisung in ein Erdgasnetz geeignete Grenzwerte für die Konzentrationen von $CO_2$ und $H_2$ aufweist. Aufgrund des umgekehrten Selektivitätsverhältnisses zwischen $\alpha 1$ und $\alpha 2$ reicht nämlich eine geringere Anzahl an Membrantrennschritten bzw. -zyklen oder auch eine geringere Membranoberfläche aus, um die $CO_2$-Konzentration unter den vorgeschriebenen Grenzwert zu bringen. Und durch den erfindungsgemäßen Einsatz der Membranen oberhalb ihrer Glasübergangstemperatur sind auch höhere Temperaturen während der Trennung möglich, was in manchen Fällen die Trenneffizienz erhöhen kann.

Der Grund, warum im Stand der Technik die Verwendung von Membranen mit solchen Selektivitätsverhältnissen für die Reinigung von Methanisierungs-Produktgasen gänzlich unbekannt ist, liegt vor allem darin, dass die Selektivitäten für Abtrennung des jeweiligen Gases $CO_2$ bzw. $H_2$ von $CH_4$ deutlich niedriger sind als jene der für diesen Zweck herkömmlicherweise eingesetzten Kunststoff- und darunter vor allem Polyimid-membranen. So weist etwa die Kunststoffmembran mit den höchsten Selektivitäten für $CO_2$ und $H_2$ unter den in den Beispielen getesteten erfindungsgemäßen Membranen nur einen Wert von 35 für $\alpha 2$ ($CO_2/CH_4$) und sogar von nur etwa 2,5 für $\alpha 1$ ($H_2/CH_4$) auf, während für Polyimidmembranen, wie oben zitiert, Werte für $\alpha 1$ ($H_2/CH_4$) von mitunter deutlich über 100 und für $\alpha 2$ ($CO_2/CH_4$) von zumindest 40 offenbart werden. Dies wird durch die Vergleichbeispiele belegt, unter denen in einem Experiment der Erfinder sogar ein $\alpha 2$-Wert von 70 erzielt wurde.

Dazu kommt aber auch, dass, wie oben erwähnt, häufig viel höhere Konzentrationen von $H_2$ im Produktgas der Methanisierung enthalten sind als von $CO_2$, speziell wenn ein Überschuss an $H_2$ in der Reaktion zum Einsatz kommt. Bei der Verwendung von Membranen, bei denen $\alpha 1/\alpha 2 < 1$ ist, gemäß vorliegender Erfindung braucht jedoch kein Überschuss, oder zumindest kein großer Überschuss, an Wasserstoff eingesetzt zu werden, da nichtumgesetztes $CO_2$ ohnehin selektiver vom erzeugten Methan abgetrennt werden kann als $H_2$ - und natürlich ebenfalls rezykliert werden kann. Das senkt die Kosten für die Rezyklierung insgesamt, da erfindungsgemäß geringere Gasmengen zu rezyklieren sind.

Wie die späteren Beispiele und Vergleichbeispiele belegen, ist gemäß vorliegender Erfindung die Abtrennung von $CO_2$ und $H_2$ von $CH_4$ mit einer signifikant höheren Energieeffizienz als nach dem Stand der Technik möglich, obwohl weitaus weniger trenneffiziente Membranen eingesetzt werden, was für den einschlägigen Fachmann höchst überraschend war.

Zwar wäre es möglich, die gemäß vorliegender Erfindung im gummiartigen Zustand oberhalb ihrer Glasübergangstemperatur arbeitenden Membranen auf erhöhte Temperaturen zu erhitzen, allerdings ist dies gar nicht erforderlich - im Gegenteil: In den Beispielen wird gezeigt, dass bei den in bevorzugten Ausführungsformen der Erfindung eingesetzten Membranen die Selektivitäten $\alpha 1$ und $\alpha 2$ für die Gastrennungen $H_2/CH_4$ bzw. $CO_2/CH_4$ mit sinkenden Temperaturen zunehmen. Gleichzeitig nimmt jedoch überraschenderweise das Selektivitätsverhältnis $\alpha 1/\alpha 2$ bei Absenkung der Betriebstempratur weiter ab. Das heißt, die Selektivität $\alpha 2$ bei der Trennung $CO_2/CH_4$ nimmt bei Annäherung an die Glasübergangstemperatur, also bei Verringerung der gummielastischen Eigenschaften der Membranen, stärker zu als die Selektivität $\alpha 1$ der Trennung $H_2/CH_4$.

Das Material der Polymer-Trennmembran ist gemäß vorliegender Erfindung nicht speziell eingeschränkt, solange es eine Glasübergangstemperatur unterhalb der jeweiligen Betriebstemperatur aufweist, d.h. bei der Betriebstemperatur im gummiartigen Zustand vorliegt, und bewirkt, dass die daraus bestehenden Membranen $CO_2$ und $H_2$ gleichzeitig von $CH_4$ abzutrennen in der Lage sind - und das mit der erfindungsgemäß höheren Selektivität für die Trennung $CO_2/CH_4$ als für die Trennung $H_2/CH_4$. Für diesen Zweck geeignete Kunststoffmembranen kann ein durchschnittlicher Fachmann mittels Gastrennungs-Routineexperimenten einfach bestimmen.

Zu den gemäß vorliegender Erfindung bevorzugten Trennmembranen, die sich in den Versuchen des Erfinders bereits bewährt haben, zählen beispielsweise solche aus Polyethern, Polyurethan-Harnstoff-Elastomeren, Polyethern, Polysiloxanen und thermoplastischen Polyether-Block-Polyamid- (PEBA-) Copolymeren, wobei besonders bevorzugt PEBA-Copolymer-Membranen eingesetzt werden, da mit diesen Membranen aufgrund des besonders niedrigen Verhältnisses zwischen $\alpha 1$ und $\alpha 2$ die obigen überraschenden Effekte reproduzierbar erzielt werden können.

Im Hinblick auf die Einspeisung des gereinigten Methans in Erdgasnetze, d.h. auf gegenwärtige und geplante zukünftige Grenzwerte, wird in bevorzugten Ausführungsformen der Erfindung im Membrantrennschritt der Gehalt an $CO_2$ im gereinigten Methan auf unter 2 Vol.-%, noch bevorzugter unter 1 Vol.-%, insbesondere unter 0,5 Vol.-%, gesenkt; und/oder der Gehalt an $H_2$ im gereinigten Methan auf unter 10 Vol.-%, unter 8 Vol.-%, unter 4 Vol.-% oder unter 2 Vol.-%, besonders bevorzugt auf unter 10 Vol.-% oder unter 8 Vol.-%, gesenkt.

Weitere bevorzugte Ausführungsformen der vorliegenden Erfindung sind aufgrund der für die erfindungsgemäß eingesetzten Membranen festgestellten Vorteile dadurch gekennzeichnet, dass die Gastrennung bei einer Betriebstemperatur $T_B$ zwischen 0 °C und 60 °C, vorzugsweise zwischen 5 °C und 30 °C, noch bevorzugter zwischen 10 °C und 25 °C durchgeführt wird. Auf diese Weise wird weiterhin oberhalb der Glasübergangstemperatur $T_g$ der Membrankunststoffe gearbeitet, es ist jedoch keine allzu aufwändige Temperatursteuerung der Trennung erforderlich, sondern es kann in besonders bevorzugten Ausführungsformen der Erfindung sogar einfach bei der jeweiligen Umgebungstemperatur im Freien gearbeitet werden - und das sogar in der kalten Jahreszeit.

Und schließlich ist es gemäß vorliegender Erfindung auch möglich, neben der Abtrennung von $CO_2$ und $H_2$ gleichzeitig auch $H_2O$ vom hergestellten Methan zu trennen, da auch die erfindungsgemäß eingesetzten Membranen für letzteren Trennschritt in der Regel die höchste Selektivität $\alpha3$ aufweisen. Auf diese Weise braucht die Vortrocknung des Produktgases der Methanisierung nicht vollständig zu erfolgen oder kann in bestimmten Situationen sogar gänzlich entfallen.

KURZBESCHREIBUNG DER ZEICHNUNG

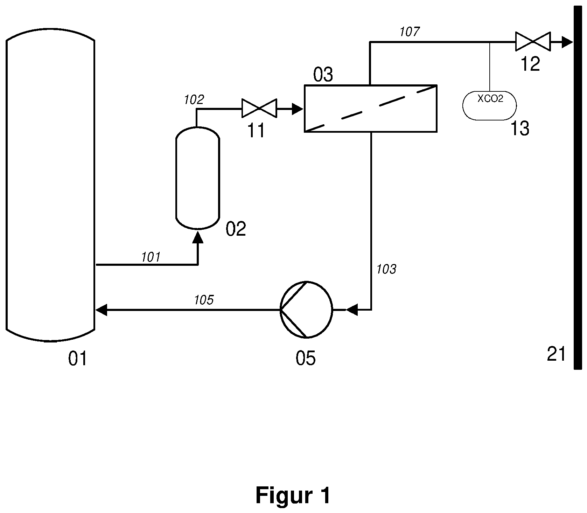

Die vorliegende Erfindung wird nachstehend anhand von nichteinschränkenden Beispielen und unter Bezugnahme auf die einzige Zeichnung näher beschrieben, wobei in Fig. 1 schematisch der Ablauf eines Verfahrens bzw. einer Anlage zur Herstellung von Methan durch Methanisierung nach dem Stand der Technik dargestellt ist, bei dem im Membrantrennschritt die erfindungsgemäßen Membranen zum Einsatz kommen.

BEISPIELE

Wie erwähnt entspricht das in Fig. 1 schematisch gezeigte Verfahren bzw. die zugehörige Anlage einer relativ einfachen Ausführungsform nach dem Stand der Technik. Dabei wird in Reaktor 01 die eigentliche Methanisierungsreaktion durch Hydrierung von - vorzugsweise aus der Umgebungsluft stammendem - Kohlenstoffdioxid gemäß der Reaktionsgleichung

$$4 H_2 + CO_2 \text{---+} CH_4 + 2 H_2O$$

durchgeführt, bei der ein an Wasser und Methan (und, wie eingangs erwähnt, gegebenenfalls weiteren Kohlenwasserstoffen, auf die nachstehend jedoch nicht näher eingegangen wird) reiches Produktgasgemisch 101 erhalten wird.

Dieses wird an Position 02 einem Vorbehandlungsschritt vor der Gastrennung unterzogen, der in der Regel eine (Vor-)Trocknung sowie gegebenenfalls eine Temperatureinstellung und/oder die Entfernung von Partikeln und anderen (z.B. aus der Umgebungsluft stammenden) für die Membranen potenziell schädlichen Komponenten, wie z.B. Ammoniak oder höheren Kohlenwasserstoffen, sowie die Beaufschlagung des Gasstroms mit dem für die Membrantrennung erforderlichen Druck umfasst. Das vorbehandelte Produktgas 102 gelangt über Regelventil 11 in den Gasmembran-Separator 03, der zumindest eine Membrantrennstufe unter Verwendung der erfindungsgemäß einzusetzenden Polymermembranen umfasst und das Gasgemisch in zumindest einen Hochdruck-Retentatstrom 107 und zumindest einen Niederdruck-Permeatstrom 103 trennt.

Aufgrund der höheren Selektivität der Membranen für die Gaskomponenten $CO_2$ und $H_2$ im Vergleich zu $CH_4$ werden $CO_2$ und $H_2$ erfindungsgemäß gleichzeitig im Permeatstrom 103 angereichert und im Retentatstrom 107 abgereichert.

Im Separator 02 werden nach dem Stand der Technik Membranen mit möglichst hohen Selektivitäten für $H_2$ und $CO_2$ in Bezug auf $CH_4$ eingesetzt, d.h. Membranen mit möglichst hohen Werten für $\alpha1$ und $\alpha2$, um pro Trennstufe möglichst große Mengen dieser beiden Gase aus dem Produktgasstrom abzutrennen. Dabei handelt es sich durchwegs um Polymermembranen, insbesondere Polyimidmembranen, im glasartigen Zustand unterhalb ihrer Glasübergangstemperatur, die allesamt eine höhere Selektivität für die Abtrennung von $H_2$ als für jene von $CO_2$ von $CH_4$, d.h. ein Verhältnis $\alpha1/\alpha2 > 1$, aufweisen. Dies ist jedoch speziell im Hinblick auf die Einspeisung des gereinigten Methans in ein Erdgasnetz nachteilig, da eine größere Anzahl an Membrantrennstufen oder -zyklen oder eine größere Membranoberfläche erforderlich ist, um den $CO_2$-Gehalt des Methans auf den zulässigen Grenzwert abzusenken. Gleichzeitig wird dabei nach dem Stand der Technik zudem die $H_2$-Konzentration auf Werte abgesenkt, die weit unter den zulässigen Grenzwerten liegen, wodurch der Rezyklat-Volumenstrom 103 unnötigerweise vergrößert wird, was höhere Energiemengen für die Rekompression durch den Kompressor 05 erfordert.

Gemäß vorliegender Erfindung werden aus diesem Grund hingegen solche Polymermembranen eingestezt, die eine höhere Selektivität für die Abtrennung von $CO_2$ als für jene von $H_2$ von $CH_4$, d.h. ein Verhältnis $\alpha1/\alpha2 < 1$, aufweisen, zumal die Grenzwerte für die $CO_2$-Konzentration, wie eingangs erwähnt, oftmals nur die Hälfte jener von $H_2$ betragen. Dadurch wird die Anzahl an erforderlichen Membrantrennschritten vor der Einspeisung in das Gasnetz deutlich verringert.

In bevorzugten Ausführungsformen umfasst der Separator gemäß vorliegender Erfindung dennoch eine Vielzahl von Membrantrennstufen der erfindungsgemäß einzusetzenden Polymermembranen, um im Retentatstrom 107, d.h. im gereinigten Methan,

den Gehalt an $CO_2$ auf unter 2 Vol.-%, noch bevorzugter unter 1 Vol.-%, insbesondere unter 0,5 Vol.-%, zu senken; und/oder

den Gehalt an $H_2$ auf unter 10 Vol.-%, unter 8 Vol.-%, unter 4 Vol.-% oder unter 2 Vol.-%, besonders bevorzugt auf unter 10 Vol.-% oder unter 8 Vol.-%, zu senken;

insbesondere beides, da auf diese Weise das gereinigte Methan im Retentat 107 ausreichend niedrige Konzentration an $CO_2$ und $H_2$ aufweist, um - nach erfolgter Konzentrationsmessung mittels Analysator 13 - über ein Regelventil 12 in ein als fettgedruckte Linie 21 dargestelltes Erdgasnetz eingespeist werden zu können.

Das Permeat 103 wird in der Folge in einem Kompressor 05 mit dem für die Methanisierung gewünschten Druck beaufschlagt und als komprimiertes Rezyklat 105 erneut in den Reaktor 01 eingeleitet.

Der Gasanalysator 13 ist aufgrund des niedrigeren Grenzwerts für $CO_2$ vorzugsweise vor allem ein $CO_2$-Analysator. Auf Basis der vom Analysator 13 bestimmten Konzentrationsmesswerten können das Regelventil 11, das Regelventil 12, der Kompressor 05 sowie die Gasvorbehandlung 02 zur Einstellung von Temperatur und/oder Druck bei Bedarf geregelt werden. Auf diese Weise kann auch das Verhältnis der Volumenströme von Retentat 107 und Permeat 103 eingestellt werden.

Im Vorbehandlungsschritt an Position 02 kann, wie oben erwähnt, auch eine Temperatureinstellung des Produktgasstroms erfolgen, um bei Bedarf die erfindungsgemäße Betriebstemperatur $T_B$ zwischen -20 °C und 100 °C oder eine erfindungsgemäß bevorzugte Betriebstemperatur zwischen 0 °C und 60 °C, noch bevorzugter zwischen 5 °C und 30 °C, insbesondere zwischen 10 °C und 25 °C, einzustellen, wobei die Grenzen jeweils eingeschlossen sind. Auf diese Weise wird gewährleistet, dass die Betriebstemperatur $T_B$ oberhalb der Glasübergangstemperatur $T_g$ der erfindungsgemäß einzusetzenden Polymermembranen liegt, wenn eine bestimmte Art von Membranen zum Einsatz kommen soll.

Die jeweilige Auswahl der Polymermembranen hängt dabei vor allem von ihrem Selektivitätsverhältnis $\alpha1/\alpha2$ und von der Zusammensetzung des jeweiligen im Reaktor 01 erzeugten Produktgasgemischs, d.h. von den Konzentrationen von $CO_2$ und $H_2$ darin, ab. Wenn beispielsweise für eine katalytische Methanisierung ein Überschuss an Wasserstoff zum Einsatz kommt und im Produktgasstrom 101 die $H_2$-Konzentration (deutlich) höher ist als jene von $CO_2$, können zur Erzielung von geeigneten $H_2$-Konzentrationen im Retentat 107 vorzugsweise solche Polymermembranen im Separator 03 eingesetzt werden, deren Selektivitätsverhältnis $\alpha1/\alpha2$ weniger weit oder sogar relativ knapp unter 1 liegt, d.h. die in der Lage sind, $CO_2$ und $H_2$ annähernd gleich gut von $CH_4$ abzutrennen. Auf diese Weise ist bei Erreichen einer für die Einspeisung in ein Erdgasnetz gemäß ÖVGW-Richtlinie G31 zulässigen $H_2$-Konzentration im Retentat 107 von beispielsweise unter 4 Vol.-% mit hoher Wahrscheinlichkeit auch die zulässige $CO_2$-Konzentration von unter 2 Vol.-% erreicht. In anderen Fällen jedoch, etwa wenn ein Überschuss an $CO_2$ für die Methanisierung zur Verfügung steht, wie dies beispielsweise bei der Gewinnung von CO2 aus der Umgebungsluft der Fall sein kann, werden erfindungsgemäß bevorzugt Membranen eingesetzt, deren Selektivitätsverhältnis $\alpha1/\alpha2$ möglichst klein ist, um pro Trennstufe deutlich mehr $CO_2$ als $H_2$ aus dem Produktgasstrom abzutrennen.

**Beispiel 1, Vergleichbeispiel 1**

Zur theoretischen Berechnung des Energieaufwands für den kontinuierlichen Betrieb einer wie in Fig. 1 gezeigt aufgebauten Anlage wurde die Durchführung eines Methanisierungsverfahrens mittels Hydrierung von $CO_2$ gemäß der Gleichung

$$4\ H_2 + CO_2 \text{ ---+ } CH_4 + 2\ H_2O$$

im Reaktor 01 mit anschließender 100%iger Trocknung des Produktgases 101 im Trockner 02 und darauf folgender Reinigung des Produktgases 102 im Separator 03 durch Abtrennung von $CO_2$ und $H_2$ von $CH_4$ mittels je einer herkömmlicherweise dafür eingesetzten Polyimidmembran im glasartigen Zustand und einer erfindungsgemäßen Polymermembran im gummiartigen Zustand, jeweils bei Umgebungstemperatur, angenommen. Weiters wurde angenommen, dass im Reaktor 01 ein Druck von 60 bar aufrechterhalten wird, um das chemische Gleichgewicht zur Produktseite hin zu verschieben, dass die Trocknung idealerweise ohne Druckverlust erfolgt und dass das an $CO_2$ und $H_2$ angereicherte Permeat 103 aus dem Separator 03 kontinuierlich zum Reaktor 01 rezykliert wird, nachdem es im Kompressor 05 wieder auf den Reaktionsdruck von 60 bar gebracht wurde. Für die Membranen wurden die nachstehenden Selektivitäten $\alpha1$ und $\alpha2$ für die Trennungen $H_2/CH_4$ ($\alpha1$) und $CO_2/CH_4$ ($\alpha2$) angenommen.

Vergleichsbeispiel 1:

Polyimidmembran (Stand der Technik): $\alpha1 = 70$ $\alpha2 = 30$ $\alpha1/\alpha2 = 2,33$

Beispiel 1:

Polyether-Block-Polyamid- (PEBA-) Membran: $\alpha1 = 2$ $\alpha2 = 20$ $\alpha1/\alpha2 = 0,10$

Diese liegen im Bereich der üblichen Selektivitäten und Selektivitätsverhältnisse für die jeweiligen Membranarten, wie die späteren Beispiele und Vergleichbeispiele belegen.

Und schließlich wurde ein maximal zulässiger $CO_2$-Gehalt im Retentat 107 von nur 0,5 Vol.-% angenommen, was zwar deutlich unter dem Grenzwert der ÖVGW-Richtlinie G31 liegt, aber im Hinblick auf für die Zukunft geplante Senkungen dieses Grenzwerts, wie eingangs erwähnt, erfolgt, um das gereinigte Methan auch nach einer solchen Senkung noch in das Erdgasnetz einspeisen zu dürfen. Gleichzeitig wird jedoch ein über jenem der Richtlinie liegender Grenzwert für den $H_2$-Gehalt angenommen, zumal geplant ist, diesen auf bis zu 10 Vol.-% zu erhöhen.

Der Unterschied im Energieverbrauch für den Betrieb des Verfahrens beruht dabei im Wesentlichen auf der Kompressionsleistung des Kompressors 05, der je nach den im Separator eingesetzten Gastrennmembranen unterschiedliche Permeat-Volumenströme zu komprimieren hat. Je höher der Druck im Reaktor, desto größer ist natürlich die Ersparnis an Kompressionsleistung durch die vorliegende Erfindung.

Die unter den obigen Annahmen berechneten Werte sind in der umseitigen Tabelle 1 zusammengefasst.

Tabelle 1

| Beschreibung | Einheit | Vergleichbeispiel 1 | Beispiel 1 |
|---|---|---|---|
| Membran-Selektivität $\alpha1$, $H_2/CH_4$ | | 70 | 2 |
| Membran-Selektivität $\alpha2$, $CO_2/CH_4$ | | 30 | 20 |
| $CO_2$-Gehalt im Methanisierungsproduktgas | [Vol.-%] | 2,0 | 2,0 |
| $H_2$-Gehalt im Methanisierungsproduktgas | [Vol.-%] | 8,0 | 8,0 |
| $CH_4$-Gehalt im Methanisierungsproduktgas | [Vol.-%] | 90,0 | 90,0 |
| Methanisierungsproduktgas-Überdruck | [bar] | 60,0 | 60,0 |
| Methanisierungsproduktgas-Volumenstrom | [Nm$^3$/h] | 6000,0 | 6000,0 |
| $CO_2$-Gehalt im Permeat | [Vol.-%] | 9,6 | 13,2 |
| $H_2$-Gehalt im Permeat | [Vol.-%] | 44,8 | 13,3 |
| $CH_4$-Gehalt im Permeat | [Vol.-%] | 45,6 | 73,5 |
| Permeat-Überdruck | [bar] | 2,0 | 2,0 |
| Permeat-Volumenstrom | [Nm$^3$/h] | 993 | 385 |
| $CO_2$-Gehalt im Retentat vor Netzeinspeisung | [Vol.-%] | 0,5 | 0,5 |
| $H_2$-Gehalt im Retentat | [Vol.-%] | 0,7 | 7,3 |
| Retentat-Volumenstrom | [Nm$^3$/h] | 5007 | 5615 |
| **Benötigte Kompressorleistung** | [kW] | **378** | **265** |
| **Verbesserung der Energieeffizienz in der Gasaufbereitung um** | **[%]** | | **30 %** |

Da die PEBA-Membran $H_2$ und $CO_2$ weniger selektiv von $CH_4$ abzutrennen in der Lage ist und daher deutlich niedrigere Absolutwerte für $\alpha1$ und $\alpha2$ ($\alpha1 = 2$, $\alpha2 = 20$) im Vergleich zu jenen der Polyimidmembran ($\alpha1 = 70$, $\alpha2 = 30$) aufweist, sind im Permeat größere Mengen an $CH_4$ enthalten (73,5 Vol.-% im Vergleich zu 45.6 Vol.-%). Dies stellt auch den Hauptgrund dafür dar, warum nach dem Stand der Technik solche Membranen bisher nicht für den erfindungsgemäßen Zweck eingesetzt wurden.

Aus der erfindungsgemäßen Gasmembrantrennung resultiert jedoch ein um mehr als die Hälfte geringerer Permeat-Volumenstrom von nur 385 Nm$^3$/h im Vergleich zu 993 Nm$^3$/h nach dem Stand der Technik, weswegen eine um 30 % geringere Kompressorleistung erforderlich ist, um das Permeat wieder mit einem Druck von 60 bar zu beaufschlagen. Bei noch höheren Drücken wäre die Energieersparnis entsprechend höher.

**Beispiele 2 bis 7, Vergleichbeispiele 2 bis 4**

In der umseitigen Tabelle 2 sind einige konkrete Membrantypen zusammen mit ihren jeweiligen Selektivitäten $\alpha1$ und $\alpha2$ und Selektivitätsverhältnissen $\alpha1/\alpha2$ angeführt, nämlich sowohl nach dem Stand der Technik bekanntermaßen für die Gasmembrantrennung eines Methanisierungs-Produktgases eingesetzte Polymermembranen im glasartigen Zustand unterhalb ihrer Glasübergangstemperatur $T_g$ als Vergleichsbeispiele 2 bis 4 (V2 bis V4) als auch gemäß vorliegender Erfindung im gummiartigen Zustand oberhalb ihrer Glasübergangstemperatur einzusetzende Polymermembranen mit umgekehrten Selektivitätsverhältnissen als Beispiele 2 bis 7 der Erfindung (B2 bis B7) angeführt.

Die Werte für $\alpha1$ und $\alpha2$ wurden dabei entweder der Fachliteratur entnommen oder vom Erfinder durch eigene Experimente bestimmt. Dazu wurden bei Raumtemperatur Reingas-Permeationsversuche mit dem jeweiligen Gas, d.h. $CH_4$, $CO_2$ oder $H_2$, bei unterschiedlichen Feedgas-Drücken durchgeführt, aus den Messergebnissen der lineare Proportionalitätsfaktor als Quotient aus dem arithmetischen Mittel der Messungen des Durchflusses bei unterschiedlichen Drücken und dem jeweiligen Druck ($m^3$/bar) berechnet und der Quotient der Proportionalitätsfaktoren für $H_2$ und $CH_4$ als $\alpha 1$ und jener der Faktoren für $CO_2$ und $CH_4$ als $\alpha 2$ für die jeweilige Membran herangezogen.

Tabelle 2

| Beispiel | Membranmaterial | Temperatur [°C] | $\alpha 1$ | $\alpha 2$ | $\alpha 1/\alpha 2$ | Quelle |
|---|---|---|---|---|---|---|
| V2 | Polyimid BPDA-arom. Diamin | 40 | 130 | 40 | 3,25 | Tanihara et al. [c] |
| V3 | Polyimid BPDA-arom. Diamin | 25 | 190 | 70 | 2,71 | Experiment |
| V4 | Polyimid 6FDA-DBBT | 35 | 80 | 45 | 1,777 | Yang et al. [d] |
| B2 | Terathane® 2900 (PolyTHF) [a] | 35 | 1,5 | 7 | 0,21 | Li et al. [e] |
| B3 | Polydimethylsiloxan (PDMS) | 23 | 1,5 | 4 | 0,375 | Experiment |
| B4 | Pebax® MH 1657 [b] | 30 | 2 | 16 | 0,125 | Car et al. [f] |
| B5 | Pebax® MH 1657 [b] | 10 | 2,5 | 26 | 0,096 | Car et al. [f] |
| B6 | Pebax® MV 1074 [b] | 27 | 2 | 16 | 0,125 | Car et al. [f] |
| B7 | PVC / Pebax® MH 1657 | 20 | 2,5 | 35 | 0,07 | Ahmadpour et al. [g] |

[a] handelsübliche Membran aus Polytetramethylenglykol-Ether (Polytetrahydrofuran, PolyTHF)
[b] handelsübliche Membranen aus Polyether-Block-Polyamid-Copolymeren (PEBA)
[c] Tanihara et al., J. Membr. Sci. 160, 179-186 (1999).
[d] Yang et al., Polymer 42, 2021 -2029 (2001).
[e] Li et al., J. Membr. Sci. 369, 49-58 (2011).
[f] Car et al., J. Membr. Sci. 307, 88-95 (2008).
[g] Ahmadpour et al., J. Nat. Gas Sci. Eng. 21, 518-523 (2014).

Aus den Ergebnissen in Tabelle 2 ist ersichtlich, dass die Selektivitätsverhältnisse $\alpha 1/\alpha 2$ der erfindungsgemäßen Polymermembranen - im Gegensatz zu den Membranen nach dem Stand der Technik im glasartigen Zustand - nicht nur unter 1 liegen, sondern darüber hinaus typischerweise um rund eine Zehnerpotenz unter jenen der herkömmlicherweise eingesetzten Membranen liegen.

Weiters zeigt ein Vergleich der Beispiele 4 und 5, dass auch für gemäß vorliegender Erfindung im gummiartigen Zustand eingesetzte Membranen die Selektivitäten für $H_2$ und $CO_2$ in Bezug auf $CH_4$, d.h. $\alpha 1$ und $\alpha 2$, mit sinkender Temperatur zunehmen, wobei $\alpha 2$ stärker zunimmt als $\alpha 1$, so dass das Selektivitätsverhältnis $\alpha 1/\alpha 2$ bei Absenkung der Betriebstempratur weiter abnimmt. Folglich sollte sich gemäß vorliegender Erfindung in der Mehrzahl der Fälle eine gezielte Erhöhung der Temperatur während der Gastrennung erübrigen.

**Beispiele 8 und 9, Vergleichbeispiele 5 bis 7**

Zur Berechnung weiterer Beispiele der vorliegenden Erfindung und Vergleichsbeispiele wurde vom analogen Betrieb einer Anlage zu jenem in Beispiel 1 und Vergleichsbeispiel 1 ausgegangen, wobei nun die oben in Tabelle 2 aufgelisteten Selektivitäten der im Handel erhältlichen Membranen der Vergleichsbeispiele 2 bis 4 und Beispiele 5 und 6 herangezogen wurden.

Die Ergebnisse sind in der umseitigen Tabelle 3 zusammengefasst.

Tabelle 3

| Beschreibung | Einheit | Vergl. 5 | Vergl. 6 | Vergl. 7 | Beispiel 8 | Beispiel 9 |
|---|---|---|---|---|---|---|
| Membran-Selektivität $\alpha 1$, $H_2/CH_4$ | | 130 | 190 | 80 | 2,5 | 2 |
| Membran-Selektivität $\alpha 2$, $CO_2/CH_4$ | | 40 | 70 | 45 | 26 | 16 |
| $CO_2$-Gehalt im Methanisierungsproduktgas | [Vol.-%] | 2,0 | 2,0 | 2,0 | 2,0 | 2,0 |
| $H_2$-Gehalt im Methanisierungsproduktgas | [Vol.-%] | 8,0 | 8,0 | 8,0 | 8,0 | 8,0 |
| $CH_4$-Gehalt im Methanisierungsproduktgas | [Vol.-%] | 90,0 | 90,0 | 90,0 | 90,0 | 90,0 |
| Methanisierungsproduktgas-Überdruck | [bar] | 60,0 | 60,0 | 60,0 | 60,0 | 60,0 |

(fortgesetzt)

| Beschreibung | Einheit | Vergl. 5 | Vergl. 6 | Vergl. 7 | Beispiel 8 | Beispiel 9 |
|---|---|---|---|---|---|---|
| Methanisierungsproduktgas-Volumenstrom | [Nm$^3$/h] | 6000,0 | 6000,0 | 6000,0 | 6000,0 | 6000,0 |
| $CO_2$-Gehalt im Permeat | [Vol.-%] | 10,0 | 11,6 | 10,9 | 13,3 | 10,3 |
| $H_2$-Gehalt im Permeat | [Vol.-%] | 48,9 | 55,3 | 49,7 | 14,2 | 12,0 |
| $CH_4$-Gehalt im Permeat | [Vol.-%] | 41,1 | 33,1 | 39,4 | 72,5 | 77,7 |
| Permeat-Überdruck | [bar] | 2,0 | 2,0 | 2,0 | 2,0 | 2,0 |
| Permeat-Volumenstrom | [Nm$^3$/h] | 946 | 812 | 864 | 702 | 915 |
| $CO_2$-Gehalt im Retentat vor Netzeinspeisung | [Vol.-%] | 0,5 | 0,5 | 0,5 | 0,5 | 0,5 |
| $H_2$-Gehalt im Retentat | [Vol.-%] | 0,34 | 0,6 | 1,0 | 7,2 | 7,3 |
| Retentat-Volumenstrom | [Nm$^3$/h] | 5054 | 5188 | 5136 | 5298 | 5085 |
| Benötigte Kompressorleistung | [kW] | **360** | **309** | **329** | **267** | **348** |

Aus den Werten für die benötigte Kompressionsleistung des Kompressors 05 ist zu erkennen, dass die gemäß vorliegender Erfindung verwendete Membran aus Beispiel 8, die - wie jene aus Beispiel 1 - ein Selektivitätsverhältnis $\alpha 1/\alpha 2$ von rund 1:10 aufwies, wiederum besser abschnitt als alle nach dem Stand der Technik regelmäßig zur Produktgasreinigung eingesetzten handelsüblichen Membranen mit umgekehrtem Selektivitätsverhältnis.

Die für das erfindungsgemäße Beispiel 9 errechnete benötigte Kompressorleistung liegt knapp über dem Durchschnitt jener der drei Vergleichsbeispiele, wobei aber in beiden erfindungsgemäßen Beispielen - bei identischem $CO_2$-Gehalt - ein $H_2$-Gehalt im gereinigten Methan erzielbar ist, der hier sogar bis zu rund 20-mal höher ist als nach dem Stand der Technik, nachdem bereits jener von Beispiel 1 mehr als das 10-fache jenes von Vergleichsbeispiel 1 betrug.

Dazu kommt außerdem, dass das sehr hohe Selektivitätsverhältnis $\alpha 1/\alpha 2$ von rund 2,7 in Vergleichsbeispiel 5 auf Labor-Messwerten der Erfinder basiert (siehe Tabelle 2, Vergleichsbeispiel 3, "Experiment"), die im praktischen Betrieb einer Gasreinigungsanlage nicht annähernd erreichbar sein wird, weswegen auch in diesem Fall signifikant größere Mengen an Permeat zu rezyklieren und erneut zu komprimieren wären, was die benötigte Kompressorleistung weiter erhöhen würde. Realistisch wäre somit für Vergleichsbeispiel 6 ein Kompressorleistungsbedarf, der etwa zwischen jenen der Vergleichsbeispiels 5 und 7 - und somit im Bereich von Beispiel 8 liegt.

### Beispiele 10 bis 17, Vergleichsbeispiele 8 bis 15

In den umseitigen Rechenbeispielen wurden unter Variation verschiedener der Verfahrensparameter jeweils paarweise Vergleiche zwischen der erfindungsgemäßen Membran aus Beispiel 8 und jener nach dem Stand der Technik aus Vergleichsbeispiel 7 angestellt, wobei wiederum ein maximaler $CO_2$-Gehalt von 0,5 Vol.-% und ein maximaler $H_2$-Gehalt von 10 Vol.-% im gereinigten Methan vorausgesetzt wurden.

Tabelle 4

| Beispiel | Membran-Selektivität | | Methanisierungs-Produktgas | | | | Permeat | | | Retentat | | Kompressor | |
|---|---|---|---|---|---|---|---|---|---|---|---|---|---|
| | $H_2/CH_4$ ($\alpha 1$) | $CO_2/CH_4$ ($\alpha 2$) | Vol.-Strom [Nm³/h] | Druck [bar] | Gehalt $CO_2$ [Vol.-%] | Gehalt $H_2$ [Vol.-%] | Druck [bar] | Gehalt $CO_2$ [Vol.-%] | Gehalt $H_2$ [Vol.-%] | Gehalt $CO_2$ [Vol.-%] | Gehalt $H_2$ [Vol.-%] | ben. Leistung [kW] | Energie-ersparnis |
| B10 | 2,5 | 26 | 6000,0 | 60,0 | 3,0 | 12,0 | 0,5 | 16,5 | 19,9 | 0,23 | 10,0 | 535 | 11 % |
| V8 | 80 | 45 | | | | | | 13,5 | 59,9 | 0,5 | 0,6 | 604 | |
| B11 | 2,5 | 26 | 6000,0 | 30,0 | 3,0 | 12,0 | 0,5 | 15,1 | 19,7 | 0,3 | 10,0 | 419 | 12 % |
| V9 | 80 | 45 | | | | | | 12,6 | 55,3 | 0,5 | 0,7 | 475 | |
| B12 | 2,5 | 26 | 6000,0 | 30,0 | 3,0 | 12,0 | 2,0 | 13,1 | 19,4 | 0,48 | 10,0 | 317 | 16 % |
| V10 | 80 | 45 | | | | | | 11,0 | 47,5 | 0,5 | 0,9 | 379 | |
| B13 | 2,5 | 26 | 6000,0 | 30,0 | 3,0 | 12,0 | 5,0 | 9,4 | 18,4 | 0,5 | 9,5 | 283 | 10 % |
| V11 | 80 | 45 | | | | | | 8,6 | 36,2 | 0,47 | 1,0 | 313 | |
| B14 | 2,5 | 26 | 6000,0 | 30,0 | 4,0 | 12,0 | 5,0 | 11,8 | 17,8 | 0,5 | 9,4 | 312 | 7 % |
| V12 | 80 | 45 | | | | | | 10,9 | 34,3 | 0,5 | 0,7 | 336 | |
| B15 | 2,5 | 26 | 6000,0 | 30,0 | 2,0 | 10,0 | 2,0 | 10,0 | 17,1 | 0,5 | 8,7 | 251 | 15 % |
| V13 | 80 | 45 | | | | | | 8,5 | 47,4 | 0,5 | 1,4 | 295 | |
| B16 | 2,5 | 26 | 6000,0 | 30,0 | 3,0 | 10,0 | 2,0 | 13,3 | 16,4 | 0,5 | 8,5 | 310 | 12 % |
| V14 | 80 | 45 | | | | | | 11,7 | 42,3 | 0,5 | 0,8 | 353 | |
| B17 | 2,5 | 26 | 6000,0 | 30,0 | 3,0 | 10,0 | 2,0 | 15,6 | 16,4 | 1,0 | 9,0 | 218 | 17 % |
| V15 | 80 | 45 | | | | | | 13,0 | 49,2 | 1,0 | 2,2 | 265 | |

Es ist zu erkennen, dass dieselbe Membran, die bei erfindungsgemäßer Verwendung in Beispiel 8 gegenüber der herkömmlichen Membran aus Vergleichsbeispiel 7 eine Kompressor-Leistungsersparnis von 11,5 % erbracht hatte, auch unter Variation diverser anderer Verfahrensparameter in den Beispielen 10 bis 17 eine Energieersparnis zwischen 7 % und 17 % bewirkt und gleichzeitig eine vor allem in Zukunft wünschenswerte Erhöhung des $H_2$-Gehalts im gereinigten Methan auf zumindest 8,5 Vol.-% mit sich bringt.

**Beispiel 18, Vergleichsbeispiel 16**

Abschließend wurden die für den Vergleich der Membranen in Beispiel 12 und Vergleichsbeispiel 10 ausgewählten Verfahrensparameter erneut herangezogen, um dieselbe Membran (siehe auch die Vergleichsbeispiele 4 und 7) mit einem Selektivitätsverhältnis $\alpha 1/\alpha 2$ von 80/45 = 1,8 sowie jene der Vergleichsbeispiele 3 und 6 mit einem Selektivitätsverhältnis $\alpha 1/\alpha 2$ von 190/70 = 2,7 mit der Membran aus Beispiel 7 zu vergleichen.

Letztere ist gemäß Ahmadpour et al. (s.o.) eine PVC/PEBA-Verbundmembran und weist ein Selektivitätsverhältnis $\alpha 1/\alpha 2$ von 2,5/35 = 0,07 und damit das niedrigste, der Literatur entnehmbare Verhältnis für die Trennungen von $H_2$ bzw. $CO_2$ von $CH_4$ auf.

Darüber hinaus wurde in diesen Vergleichen jedoch keine fixe Obergrenze für den $H_2$-Gehalt im gereinigten Methan voreingestellt.

Die Ergebnisse sind umseitig in Tabelle 5 zusammengefasst.

Tabelle 5

| Beispiel | Membran-Selektivität | | Methanisierungs-Produktgas | | | | Permeat | | | Retentat | | Kompressor | |
|---|---|---|---|---|---|---|---|---|---|---|---|---|---|
| | $H_2/CH_4$ ($\alpha$1) | $CO_2/CH_4$ ($\alpha$2) | Vol.-Strom [Nm³/h] | Druck [bar] | Gehalt $CO_2$ [Vol.-%] | Gehalt $H_2$ [Vol.-%] | Druck [bar] | Gehalt $CO_2$ [Vol.-%] | Gehalt $H_2$ [Vol.-%] | Gehalt $CO_2$ [Vol.-%] | Gehalt $H_2$ [Vol.-%] | ben. Leistung [kW] | Energie-ersparnis |
| B18 | 2,5 | 35 | 6000,0 | 30,0 | 3,0 | 12,0 | 2,0 | 14,8 | 19,2 | 0,5 | 10,5 | 277 | **27 %** |
| V10 | 80 | 45 | | | | | | 11,0 | 47,5 | 0,5 | 0,9 | 379 | |
| B18 | 2,5 | 35 | 6000,0 | 30,0 | 3,0 | 12,0 | 2,0 | 14,8 | 19,2 | 0,5 | 10,5 | 277 | **23 %** |
| V16 | 190 | 70 | | | | | | 11,5 | 51,1 | 0,5 | 0,5 | 360 | |

Es ist klar ersichtlich, dass die Energieersparnis aufgrund der reduzierten erforderlichen Kompressorleistung in diesen Fällen deutlich höher ausfiel als oben in Tabelle 4 im Falle der erfindungsgemäßen Verwendung der Membran mit einem Selektivitätsverhältnis $\alpha 1/\alpha 2$ von 2,5/26 = 0,1, nämlich um weitere 50 % besser als davor.

Dies brachte zwar einen $H_2$-Gehalt im gereinigten Methan von 10,5 Vol.-% mit sich, allerdings ist klar, dass auch bei einer Limitierung desselben auf 10,0 Vol.-% die Ergebnisse kaum schlechter gewesen wären.

Daraus folgt für den durchschnittlichen Fachmann, dass mit der Entwicklung von Polymer-Membranen, wie z.B. Elastomer-Membranen, mit noch niedrigeren Selektivitätsverhältnissen $\alpha 1/\alpha 2$ die vorliegende Erfindung mit hoher Wahrscheinlichkeit eine noch höhere Energieeffizienz bei der Reinigung der Produktgase von Methanisierungen ermöglichen wird.

Weitere Experimente zur Bestimmung anderer gemäß vorliegender Erfindung geeigneter Gastrennmembranen analog zu den oben beschriebenen sind jedenfalls derzeit Gegenstand der Forschungen des Erfinders.

Die vorliegende Erfindung stellt somit ein neues Verfahren zur Herstellung von Methan durch Methanisierung und anschließenden Reinigung mittels Gasmembrantrennung bereit, das nicht nur, aber vor allem dann große Vorteile gegenüber den Verfahren nach dem Stand der Technik aufweist, wenn sehr niedrige Grenzwerte für die Konzentration von $CO_2$ im gereinigten Methan einzuhalten sind.

**Patentansprüche**

1. Verwendung von Polymer-Trennmembranen, die in der Lage sind, $CO_2$ und $H_2$ selektiv von $CH_4$ abzutrennen, in einem Membrantrennschritt zur Reinigung von in einem $CH_4$, $H_2$ und $CO_2$ umfassenden, gegebenenfalls vorgetrockneten, Produktgasgemisch eines Methanisierungsverfahrens enthaltenem Methan,
**dadurch gekennzeichnet, dass**

    a) die Trennung bei einer Betriebstemperatur $T_B$ zwischen -20 °C und 100 °C durchgeführt wird; und
    b) die Polymermembranen

        b1) in der Lage sind, $CO_2$ und $H_2$ gleichzeitig von $CH_4$ abzutrennen,
        b2) eine höhere Selektivität für die Abtrennung von $CO_2$ als für jene von $H_2$ von $CH_4$, d.h. ein Verhältnis $\alpha 1/\alpha 2 < 1$, aufweisen und
        b3) eine Glasübergangstemperatur $T_g$ aufweisen, die unter der Betriebstemperatur $T_B$ liegt.

2. Verfahren zur Produktion von Methan, das die folgenden Schritte umfasst:

    einen Methanisierungsschritt, in dem durch Reduktion von $CO_2$ mit $H_2$ ein neben $CH_4$ auch $H_2O$, $H_2$ und $CO_2$ umfassendes Produktgas gebildet wird;
    gegebenenfalls einen Trockungsschritt, in dem $H_2O$ aus dem Produktgas entfernt wird; und
    einen Membrantrennschritt zur Reinigung des Methans, in dem das durch die Trocknung erhaltene, $CH_4$, $H_2$ und $CO_2$ umfassende Gasgemisch einer Trennung unter Verwendung von Trennmembranen unterzogen wird, die in der Lage sind, $CO_2$ und $H_2$ selektiv von $CH_4$ abzutrennen;

    **dadurch gekennzeichnet, dass**

    a) die Trennung im Membrantrennschritt bei einer Betriebstemperatur $T_B$ zwischen -20 °C und 100 °C durchgeführt wird; und
    b) Polymermembranen eingesetzt werden, die

        b1) in der Lage sind, $CO_2$ und $H_2$ gleichzeitig von $CH_4$ abzutrennen,
        b2) eine höhere Selektivität für die Abtrennung von $CO_2$ als für jene von $H_2$ von $CH_4$, d.h. ein Verhältnis $\alpha 1/\alpha 2 < 1$, aufweisen und
        b3) eine Glasübergangstemperatur $T_g$ aufweisen, die unter der Betriebstemperatur $T_B$ liegt.

3. Verwendung nach Anspruch 1 oder Verfahren nach Anspruch 2, **dadurch gekennzeichnet, dass** im Membrantrennschritt

    der Gehalt an $CO_2$ im gereinigten Methan auf unter 2 Vol.-%, unter 1 Vol.-% oder unter 0,5 Vol.-% gesenkt wird; und/oder
    der Gehalt an $H_2$ im gereinigten Methan auf unter 10 Vol.-%, unter 8 Vol.-%, unter 4 Vol.-% oder unter 2 Vol.-

% gesenkt wird.

4. Verwendung oder Verfahren nach einem der Ansprüche 1 bis 3, **dadurch gekennzeichnet, dass** als Trennmembranen solche aus Polyethern, Polyurethan-Harnstoff-Elastomeren, Polysiloxanen oder thermoplastischen Polyether-Block-Polyamid-(PEBA-) Copolymeren eingesetzt werden.

5. Verwendung oder Verfahren nach Anspruch 3, **dadurch gekennzeichnet, dass** als Trennmembranen PEBA-Copolymer-Membranen eingesetzt werden.

6. Verwendung oder Verfahren nach einem der Ansprüche 1 bis 5, **dadurch gekennzeichnet, dass** die Trennung bei einer Betriebstemperatur $T_B$ zwischen 0 °C und 60 °C durchgeführt wird.

7. Verwendung oder Verfahren nach Anspruch 6, **dadurch gekennzeichnet, dass** die Trennung bei einer Betriebstemperatur $T_B$ zwischen 5 °C und 30 °C durchgeführt wird.

8. Verwendung oder Verfahren nach Anspruch 7, **dadurch gekennzeichnet, dass** die Trennung bei einer Betriebstemperatur $T_B$ zwischen 10 °C und 25 °C durchgeführt wird.

9. Verwendung oder Verfahren nach einem der Ansprüche 1 bis 8, **dadurch gekennzeichnet, dass** die Trennmembranen in der Lage sind, neben $CO_2$ und $H_2$ gleichzeitig auch Reste von $H_2O$ von $CH_4$ abzutrennen.

**Claims**

1. A use of polymer separation membranes being able to selective separate $CO_2$ and $H_2$ from $CH_4$ in a membrane separation step for purifying methane contained in an optionally pre-dried product gas mixture of a methanation method, which comprises $CH_4$, $H_2$ and $CO_2$,
**characterized in that**

   a) the separation is carried out at an operation temperature $T_B$ between -20 °C and 100 °C; and
   b) the polymer membranes

   b1) are able to simultaneously separate $CO_2$ and $H_2$ from $CH_4$,
   b2) have a higher selectivity for the separation of $CO_2$ than of $H_2$ from $CH_4$, i.e., a ratio $\alpha1/\alpha2 < 1$, and
   b3) have a glass transition temperature $T_g$ that is lower than the operation temperature $T_B$.

2. A method for producing methane, comprising the following steps:

   a methanation step in which, by reducing $CO_2$ with $H_2$, a product gas is formed that comprises $H_2O$, $H_2$ and $CO_2$ in addition to $CH_4$;
   optionally a drying step in which $H_2O$ is removed from the product gas; and
   a membrane separation step for purifying the methane, wherein the gas mixture obtained by drying and containing $CH_4$, $H_2$ and $CO_2$ is subjected to separation using separation membranes being able to selectively separate $CO_2$ and $H_2$ from $CH_4$;

   **characterized in that**

   a) the separation in the membrane separation step is conducted at an operation temperature $T_B$ between -20 °C and 100 °C; and
   b) polymer membranes are used that

   b1) are able to simultaneously separate $CO_2$ and $H_2$ from $CH_4$,
   b2) have a higher selectivity for the separation of $CO_2$ than of $H_2$ from $CH_4$, i.e., a ratio $\alpha1/\alpha2 < 1$, and
   b3) have a glass transition temperature $T_g$ that is lower than the operation temperature $T_B$.

3. The use according to claim 1 or the method according to claim 2, **characterized in that** in the membrane separation step,

the content of $CO_2$ in the purified methane is lowered to below 2 vol%, below 1 vol% or below 0.5 vol%; and/or the content of $H_2$ in the purified methane is lowered to below 10 vol%, below 8 vol%, below 4 vol%, or below 2 vol%.

4. The use or the method according to any one of claims 1 to 3, **characterized in that** the separation membranes used are those made of polyethers, poly(urethane-urea) elastomers, polyethers, polysiloxanes, and thermoplastic polyether-block-polyamide (PEBA) copolymers.

5. The use or the method according to claim 3, **characterized in that** the separation membranes used are PEBA copolymer membranes.

6. The use or the method according to any one of claims 1 to 5, **characterized in that** the separation is conducted at an operation temperature $T_B$ between 0 °C and 60 °C.

7. The use or the method according to claim 6, **characterized in that** the separation is conducted at an operation temperature $T_B$ between 5 °C and 30 °C.

8. The use or the method according to claim 7, **characterized in that** the separation is conducted at an operation temperature $T_B$ between 10 °C and 25 °C.

9. The use or the method according to any one of claims 1 to 8, **characterized in that** the separation membranes are able to, in addition to $CO_2$ and $H_2$, simultaneously also separate residual amounts of $H_2O$ from $CH_4$,

**Revendications**

1. Utilisation de membranes polymères de séparation susceptibles de séparer sélectivement le $CO_2$ et le $H_2$ du $CH_4$ dans une étape de séparation par les membranes pour purifier du méthane contenu dans un mélange, éventuellement préséché, d'un produit gazeux d'un procédé de méthanisation, le mélange comprenant du $CH_4$, de l'$H_2$ et du $CO_2$, **caractérisée en ce que**

   a) la séparation est effectuée à une température de fonctionnement $T_B$ comprise entre -20 °C et 100 °C ; et
   b) les membranes polymères

   b1) sont susceptibles de séparer simultanément le $CO_2$ et le $H_2$ du $CH_4$,
   b2) ont une sélectivité plus élevée pour la séparation de $CO_2$ par rapport à la séparation de l'$H_2$ du $CH_4$, c'est-à-dire un rapport de $\alpha 1/\alpha 2 < 1$, et
   b3) ont une température de transition vitreuse $T_g$ qui est inférieure à la température de fonctionnement $T_B$.

2. Procédé pour la production de méthane, comprenant les étapes suivantes :

   une étape de méthanisation, dans laquelle est formé un produit gazeux par la réduction de $CO_2$ avec le $H_2$, le produit gazeux comprenant, en plus de $CH_4$, de l'$H_2O$, de l'$H_2$ et du $CO_2$ ;
   éventuellement une étape de séchage, dans laquelle le $H_2O$ est éliminé du produit gazeux ; et
   une étape de séparation par des membranes pour purifier le méthane, dans laquelle le mélange gazeux obtenu par le séchage et comprenant du $CH_4$, de l'$H_2$ et du $CO_2$ est soumis à séparation par des membranes de séparation susceptibles de séparer sélectivement le $CO_2$ et le $H_2$ du $CH_4$ ;

   **caractérisé en ce que**

   a) la séparation dans l'étape de séparation par les membranes est effectuée à une température de fonctionnement $T_B$ comprise entre -20 °C et 100 °C ; et
   b) des membranes polymères sont utilisées, qui

   b1) sont susceptibles de séparer simultanément le $CO_2$ et le $H_2$ du $CH_4$,
   b2) ont une sélectivité plus élevée pour la séparation de $CO_2$ par rapport à la séparation de l'$H_2$ du $CH_4$, c'est-à-dire un rapport de $\alpha 1/\alpha 2 < 1$, et
   b3) ont une température de transition vitreuse $T_g$ qui est inférieure à la température de fonctionnement $T_B$.

3. Utilisation selon la revendication 1 ou procédé selon la revendication 2, caractérisé(e) en ce que dans l'étape de séparation par des membranes

la teneur en $CO_2$ dans le méthane purifié est réduit à moins de 2 % en volume, moins de 1 % en volume ou moins de 0,5 % en volume; et/ou
la teneur en $H_2$ dans le méthane purifié est réduit à moins de 10 % en volume, moins de 8 % en volume ou moins de 2 % en volume.

4. Utilisation ou procédé selon l'une quelconque des revendications 1 à 3, caractérisé(e) en ce que comme membranes de séparation sont utilisées des membranes en polyéthers, en élastomères de polyuréthane-urée, en polysiloxanes ou en copolymères thermoplastiques de polyéther bloc amide (PEBA).

5. Utilisation ou procédé selon la revendication 3, caractérisé(e) en ce que comme membranes de séparation sont utilisées de membranes en copolymères de PESA.

6. Utilisation ou procédé selon l'une quelconque des revendications 1 à 5, caractérisé(e) en ce que la séparation est effectuée à une température de fonctionnement $T_B$ comprise entre 0 °C et 60 °C.

7. Utilisation ou procédé selon la revendication 6, caractérisé(e) en ce que la séparation est effectuée à une température de fonctionnement $T_B$ comprise entre 5 °C et 30 °C.

8. Utilisation ou procédé selon la revendication 7, caractérisé(e) en ce que la séparation est effectuée à une température de fonctionnement $T_B$ comprise entre 10 °C et 25 °C.

9. Utilisation ou procédé selon l'une quelconque des revendications 1 à 8, caractérisé(e) en ce que les membranes de séparation sont susceptibles de séparer, en plus de $CO_2$ et $H_2$, des restes de $H_2O$ et de $CH_4$ du $CH_4$

**Figur 1**

**EP 3 988 633 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2015017875 A1

**In der Beschreibung aufgeführte Nicht-Patentliteratur**

- **TANIHARA et al.** *J. Membr. Sci,* 1999, vol. 160, 179-186
- **YANG et al.** *Polymer,* 2001, vol. 42, 2021-2029
- **LI et al.** *J. Membr. Sci,* 2011, vol. 369, 49-58
- **CAR et al.** *J. Membr. Sci,* 2008, vol. 307, 88-95
- **CAR et al.** *Sep. Purif. Technol.,* 2008, vol. 62, 110-117
- **AHMADPOUR et al.** *J. Nat. Gas Sci. Eng,* 2014, vol. 21, 518-523
- **TANIHARA et al.** *J. Membr. Sci.,* 1999, vol. 160, 179-186
- **YANG et al.** *Polymer,* 2001, vol. 42, 2021-2029